(19) Europäisches Patentamt
European Patent Office
Office européen des brevets

(11) Veröffentlichungsnummer: **0 392 362 A2**

(12) # EUROPÄISCHE PATENTANMELDUNG

(21) Anmeldenummer: **90106531.8**

(51) Int. Cl.$^5$: **B01D 67/00, C08J 7/06**

(22) Anmeldetag: **05.04.90**

(30) Priorität: **11.04.89 DE 3911864**

(43) Veröffentlichungstag der Anmeldung: **17.10.90 Patentblatt 90/42**

(84) Benannte Vertragsstaaten: **AT BE CH DE FR GB IT LI NL SE**

(71) Anmelder: **FhG**
**FRAUNHOFER-GESELLSCHAFT ZUR FÖRDERUNG DER ANGEWANDTEN FORSCHUNG E.V.**
**Leonrodstrasse 54**
**D-8000 München 19(DE)**

(72) Erfinder: **Gröbe, Anneliese, Dr.**
**Filderstrasse 64**
**D-7300 Esslingen(DE)**
Erfinder: **Chmiel, Horst, Prof., Dr.**
**Paracelsusstrasse 24**
**D-7250 Leonberg(DE)**

(54) **Verfahren zur Oberflächenmodifizierung von Polymer-Membranen.**

(57) Die Erfindung betrifft ein Verfahren zur Oberflächenmodifizierung von Polymer-Membranen, um deren Anti-Fouling-Verhalten gegenüber Proteinen zu verbessern. Die Polymer-Membranen werden dazu mit organisch modifizierten Silanen der allgemeinen Formel (I)

```
     R'
     |
  X-Si-Y        (I),
     |
     Y
```

worin
X = H, Alkyl, Aryl oder Aralkyl, jeweils mit $C_1$ bis $C_{20}$,
R′ = Alkyl, Aryl, Aralkyl, jeweils mit $C_1$ bis $C_{20}$, oder Y, und
Y = OR, OOCR oder NRR″, mit R = Alkyl, Aryl oder Aralkyl, jeweils mit $C_1$ bis $C_{20}$, und R″ = H oder R, bedeuten,
oder mit Gemischen dieser organisch modifizierten Silane (I) beschichtet. Die so modifizierten Polymer-Membranen zeigen gegenüber den unbehandelten Membranen nach Kontakt mit Proteinen eine stark verminderte Abnahme des Transmembranenflusses.

EP 0 392 362 A2

## Verfahren zur Oberflächenmodifizierung von Polymer-Membranen

Die Erfindung betrifft ein Verfahren zur Oberflächenmodifizierung von Polymer-Membranen, um deren Anti-Fouling-Verhalten gegenüber Proteinen zu verbessern.

Im medizinischen und biologischen Bereich werden sowohl in der Forschung als auch in der routinemäßigen Prüfung Flüssigkeiten, welche proteinhaltiges Material als feste Partikel oder in gelöster bzw. gequollener Form enthalten, über Membrantrennprozesse untersucht bzw. aufbereitet. Ähnliche Systeme werden in der biotechnologischen Verfahrenstechnik ebenfalls über Membrantrennprozesse aufgearbeitet. Beispiele für solche Trennverfahren mittels Membranen wären etwa die Ultra- oder Mikrofiltration, die Dialyse, die Osmose oder die Reversosmose. Erwähnt werden sollen in diesem Zusammenhang auch die zahlreichen Enzymelektroden.

Obwohl die bei diesen Trennverfahren eingesetzten Membranen entsprechend dem jeweiligen Anwendungsfall aus unterschiedlichen Materialien bestehen und bzgl. ihrer Struktur sehr unterschiedlich aufgebaut sind, verfügen sie jedoch alle aufgrund ihres Fouling-Verhaltens nur über eine begrenzte Einsetzbarkeit bei proteinhaltigen Flüssigkeiten. Unter Fouling versteht man eine nichtspezifische Proteinadsorption an den Membranen, welche deren Permeabilität drastisch verringert.

Besonders anfällig für derartige Ablagerungen, vorallem an den Oberflächen, sind hydrophobe Membranen mit schlechter Benetzbarkeit, wie z.B. solche aus Polysulfonen oder aus Polyvinylidenfluorid. Vorteil dieser Polymere ist jedoch ihre thermische Stabilität, die garantiert, daß Membranen aus diesen Materialien gut sterilisierbar sind.

Da Membranen aus hydrophilen Polymeren, wie z.B. aus Cellulosederivaten, ein etwas verbessertes Anti-Fouling-Verhalten zeigen, hat man versucht, hydrophobe Polymere zu hydrophilisieren, z.B. durch Zusatz von hydrophilen Polymeren wie etwa Polyvinylacetat oder Polyvinylpyrrolidon zu Polyvinylidenfluorid (EP 0012557 A1). Um jedoch gesteigerte hydrophile Effekte zu erreichen, müssen entweder so große Mengen an Polyvinylpyrrolidon zugesetzt werden, daß die mechanische Beständigkeit der resultierenden Membranen herabgesetzt wird, oder aber das Polyvinylacetat ist in einem zweiten aufwendigen Verfahrensschritt zu verseifen.

Ferner hat man versucht, das Anti-Fouling-Verhalten von Polymermembranen dadurch zu verbessern, daß man diese Membranen mit modifizierten Halogensilanen, speziell mit Chlorsilanen, wie z.B. Phenyltrichlorsilan, Dimethyldichlorsilan oder Methyltrichlorsilan, behandelt (EP 0204468 A2). Diese Silane hydrolisieren aber sehr rasch und zwar bereits bei Anwesenheit von Luftfeuchtigkeit unter Abspaltung von Halogenwasserstoff - bei Einsatz von Chlorsilanen unter Freisetzung von Chlorwasserstoff - welcher die Membranen angreift und brüchig werden läßt. Außerdem werden bei der Hydrolyse dieser Silane Protonen freigesetzt, welche im Rahmen einer Autokatalyse die unkontrollierte Vernetzung dieser Silane verursachen. Diese Vernetzung ist nicht steuerbar, es entsteht ein 3-dimesionales Netzwerk mit hohen Vernetzungsgraden, die Filme werden spröde.

Aufgabe der Erfindung ist es nun, ein Verfahren bereitzustellen, mit dem die Oberfläche von Polymermembranen derart modifiziert werden kann, daß deren Anti-Fouling-Verhalten drastisch verbessert wird, ohne jedoch gleichzeitig deren physikalischen Eigenschaften, wie etwa deren Stabilität, nachteilig zu verändern. Außerdem soll dieses Verfahren unabhängig vom Polymermaterial und unabhängig von der Struktur der Membranen anzuwenden sein.

Überraschenderweise wurde nun gefunden, daß man zu einer drastischen Verbesserung des Anti-Fouling-Verhaltens von Polymer-Membranen kommt, wenn man diese mit organisch modifizierten Silanen der allgemeinen Formel (I) oder mit Gemischen dieser Silane (I) behandelt,

$$\begin{array}{c} R' \\ | \\ X-Si-Y \\ | \\ Y \end{array} \qquad (I)$$

worin
X = H, Aklyl, Aryl oder Aralkyl, jeweils mit $C_1$ bis $C_{20}$,
R′ = Alkyl, Aryl, Aralkyl, jeweils mit $C_1$ bis $C_{20}$, oder Y, und
Y = OR, OOCR oder NRR″, mit R = Alkyl, Aryl oder Aralkyl, jeweils mit $C_1$ bis $C_{20}$, und R″ = H oder R, bedeuten.

Das erfindungsgemäße Verfahren ist unabhängig vom Polymer-Material und unabhängig von der

Struktur der zubehandelnden Polymer-Membranen anwendbar. Mit dem erfindungsgemäßen Verfahren können sowohl hydrophobe als auch hydrophile Membranen modifiziert werden. Die zubehandelnden Membranen können z.B. aus Cellulose-Polymeren oder aus Polyvinylidenfluorid bestehen. Es können ferner Kompositmembranen, wie z.B. mit sulfonierten Polysulfonen beschichtete Polysulfon-Membranen, behandelt werden. Die zu behandelnden Membranen können z.B. durch Schmelzextrusion oder durch Phaseninversion gebildet worden sein. Ferner können prinzipiell alle handelsüblichen Membranen, die mit den allgemein üblichen Konditionierungsmitteln, wie z.B. Glycerin oder Polyethylen glycol, ausgestattet wurden mit dem erfindungsgemäßen Verfahren derart modifiziert werden, daß diese ein drastisch verbessertes Anti-Fouling-Verhalten zeigen.

Die organisch modifizierten Silane der allgemeinen Formel (I) bzw. deren Mischungen können in dem erfindungsgemäßen Verfahren mit oder ohne Lösungsmittel eingesetzt werden. So ist z.B. das reine Triethoxysilan ebenso einsetzbar wie dasjenige, welches in Petrolether (Mischungsverhältnis z.B. 1:1) gelöst ist.

Die organisch modifizierten Silane der Formel (I) bzw. deren Mischungen oder die Lösungen dieser Silane (I) bzw. die Lösungen der Gemische können in dem erfindungsgemäßen Verfahren auf die Polymer-Membranen einmal oder mehrmals aufgesprüht bzw. aufgestrichen werden, oder aber die mit dem erfindungsgemäßen Verfahren zu behandelnden Polymer-Membranen können in die organisch modifizierten Silane der Formel (I) bzw. in deren Mischungen oder in die Lösungen dieser Silanen bzw. in die Lösungen der Gemische einmal oder mehrmals getaucht werden.

Anhand der folgenden Beispiele soll die Brauchbarkeit des erfindungsgemäßen Verfahrens demonstriert werden. Dazu wurde der Wasserfluß von handelsüblichen Polymer-Membranen und von nach dem erfindungsgemäßen Verfahren modifizierten Polymer-Membranen bestimmt, deren Rückhaltevermögen für Albumin (aus Rinderserum, MG≈67000, Produkt der Firma Fluka, Best.-Nr. 05470) bzw. Cytochrom C (aus Pferdeherz, MG≈13000, Produkt der Firma Fluka, Best.-Nr. 30400) ermittelt und erneut der Wasserfluß bestimmt. Die Bestimmung des Wasserflusses erfolgte mit bidestilliertem Wasser in einer Berghofzelle mit einem Druck von 1 bar. Dabei wurde diejenige Wassermenge ermittelt, die pro Zeiteinheit die Grundfläche (76 $mm^2$) des Filters passiert. Die Einheit des Wasserflusses ist $l/m^2hbar$. Es wurde stets frische Lösungen von Albumin (0.1 %ig in Phosphatpuffer, pH = 7.4) und von Cytochrom C (0.01 %ig in Phosphatpuffer, pH = 7.2) eingesetzt. Die Konzentrationen der Stammlösungen, der Filtrate (nach 50 ml Vorlauf) und der Konzentrate wurden photometrisch für Albumin bei 278 nm und für Cytochrom C bei 408 nm bestimmt. Für das Rückhaltevermögen R (in %) gilt:

$$R[\%] = 1 - \frac{\text{Extinktion Filtrat}}{\text{Extinktion Konzentrat}}$$

Beispiel 1:

Der Wasserfluß einer handelsüblichen Kompositmembran (Polysulfon-Membran mit sulfoniertem Polysulfon beschichtet) wurde an je vier Beispielen zweimal bestimmt. Danach wurde deren Rückhaltevermögen für das Albumin ermittelt und anschließend erneut der Wasserfluß bestimmt. Es war eine drastische Abnahme des Wasserflusses zu verzeichnen.

| Wasserfluß d. Membran [1/$m^2$hbar] | Rückhaltevermögen f. Albumin | veränderter Wasserfluß [1/$m^2$hbar] | Abnahme d.Wasserflusses |
|---|---|---|---|
| 17.7(19.2) | 99.7 % | 11.2 | 36.8 % |
| 17.5(20.6) | 98.8 % | 13.3 | 24.0 % |
| 19.8(20.8) | 98.9 % | 14.4 | 27.3 % |
| 19.8(21.7) | 98.9 % | 14.4 | 27.3 % |

Vier weitere Exemplare dieses Membrantyps wurden nun nach dem erfindungsgemäßen Verfahren 4-mal mit Triethoxysilan (unverdünnt, ohne Lösungsmittel) beschichtet. Von diesen modifizierten Membranen

wurde dann wie oben beschrieben der Wasserfluß, das Rückhaltevermögen für das Albumin und der veränderte Wasserfluß bestimmt. Es war nur eine geringfügige Veränderung des Wasserflusses zu verzeichnen

| Wasserfluß d. Membran [$1/m^2hbar$] | Rückhaltevermögen f. Albumin | veränderter Wasserfluß [$1/m^2hbar$] | Abnahme d.Wasserflusses |
|---|---|---|---|
| 13.9 | 98.8 % | 13.3 | 4.4 % |
| 15.5 | 99.9 % | 14.3 | 7.8 % |
| 15.0 | 99.8 % | 14.3 | 4.7 % |
| 17.7 | 99.9 % | 16.3 | 7.9 % |

Beispiel 2:

Bei einer modifizierten Polyvinylidenfluorid-Membran (Fällungsmittel : $H_2O$, Trenngrenze : MG = 10000) wurde an vier Exemplaren der Wasserfluß, das Rückhaltevermögen für das Albumin und der veränderte Wasserfluß bestimmt. Es war eine extrem starke Abnahme des Wasserflusses zu verzeichnen.

| Wasserfluß d. Membran [$1/m^2hbar$] | Rückhaltevermögen f. Albumin | veränderter Wasserfluß [$1/m^2hbar$] | Abnahme d.Wasserflusses |
|---|---|---|---|
| 591.8 | 99.8 % | 108.2 | 81.8 % |
| 489.4 | 98.5 % | 79.1 | 84.0 % |
| 611.5 | 95.8 % | 57.6 | 90.6 % |
| 501.3 | 98.5 % | 69.3 | 86.2 % |

Nun wurden vier weitere Exemplare dieses Membrantyps nach dem erfindungsgemäßen Verfahren mit Triethoxysilan (verdünnt mit Petrolether (Siedefraktion : 40-70 °C) im Verhältnis 1:1) behandelt. Von den so modifizierten Membranen wurde dann wie oben beschrieben der Wasserfluß, das Rückhaltevermögen für Albumin und der veränderte Wasserfluß beschrieben. Es wurde eine deutlich verminderte Abnahme des Wasserflusses verzeichnet.

| Wasserfluß d. Membran [$1/m^2hbar$] | Rückhaltevermögen f. Albumin | veränderter Wasserfluß [$1/m^2hbar$] | Abnahme d.Wasserflusses |
|---|---|---|---|
| 242.5 | 80.0 % | 200.5 | 17.4 % |
| 279.2 | 99.4 % | 145.4 | 48.0 % |
| 177.8 | 99.4 % | 117.4 | 34.0 % |
| 192.5 | 99.4 % | 134.4 | 32.0 % |

Vier weitere Exemplare dieses Membrantyps wurden nach dem erfindungsgemäßen Verfahren 3-mal mit Triethoxysilan (unverdünnt, ohne Lösungsmittel) beschichtet. Von den so modifizierten Membranen wurde dann wieder des Wasserfluß, das Rückhaltevermögen für das Albumin und der veränderte Wasserfluß bestimmt. Es war nur eine geringfügige Abnahme des Wasserflußes zu verzeichnen.

| Wasserfluß d. Membran [1/m²hbar] | Rückhaltevermögen f. Albumin | veränderter Wasserfluß [1/m²hbar] | Abnahme d.Wasserflusses |
|---|---|---|---|
| 32.4 | 99.1 % | 31.3 | 3.4 % |
| 49.5 | 98.7 % | 41.2 | 16.8 % |
| 50.4 | 98.2 % | 39.7 | 21.3 % |
| 30.3 | 94.2 % | 30.7 | 0 % |

Vier weitere Exemplare dieses Membrantyps wurden 2-mal mit Triethoxysilan (unverdünnt, ohne Lösungsmittel) beschichtet. Anschließend wurde der Wasserfluß, das Rückhaltevermögen für das Albumin und der veränderte Wasserfluß bestimmt. Es war nur eine geringfügige Veränderung des Wasserflusses zu verzeichnen.

| Wasserfluß d.Membran [1/m²hbar] | Rückhaltevermögen f. Albumin | veränderter Wasserfluß [1/m²hbar] | Abnahme d.Wasserflusses |
|---|---|---|---|
| 44.9 | 97.4 % | 44.8 | 0.3 % |
| 43.8 | 98.8 % | 41.6 | 5.1 % |
| 38.8 | 98.3 % | 34.2 | 11.9 % |
| 38.7 | 98.4 % | 36.8 | 5.0 % |

Beispiel 3:

An vier Exemplaren einer modifizierten Polyvinylidenfluorid-Membran (Fällungsmittel : Wasser, Trenngrenze : MG = 2000) wurde der Wasserfluß, anschließend das Ruckhaltevermögen für das Cytochrom C und der veränderte Wasserfluß bestimmt. Es war eine drastische Abnahme des Wasserflusses zu verzeichnen.

| Wasserfluß d. Membran [1/m²hbar] | Rückhaltevermögen f. Cytochrom C | veränderter Wasserfluß [1/m²hbar] | Abnahme d.Wasserflusses |
|---|---|---|---|
| 26 | 100 % | 4 | 84.7 % |
| 35 | 100 % | 5 | 85.8 % |
| 35 | 100 % | 5 | 85.8 % |
| 30 | 100 % | 4 | 86.7 % |

Diese Versuchsreihe wurde wiederholt.

| Wasserfluß d. Membran [1/m²hbar] | Rückhaltevermögen f. Cytochrom C | veränderter Wasserfluß [1/m²hbar] | Abnahme d.Wasserflusses |
|---|---|---|---|
| 37 | 99.8 % | 8 | 88.4 % |
| 44 | 100 % | 8 | 81.8 % |
| 35 | 100 % | 6 | 82.9 % |
| 31 | 100 % | 5 | 83.9 % |

Vier weitere Exemplare dieses Membrantyps wurden 2-mal mit Triethoxysilan (unverdünnt, ohne

Lösungsmittel) beschichtet. Anschließend wurde der Wasserfluß, das Rückhaltevermögen für das Cyctochrom C und der veränderte Wasserfluß bestimmt. Es war nur eine geringfügige Abnahme des Wasserflusses zu verzeichnen.

| Wasserfluß d. Membran [1/m²hbar] | Rückhaltevermögen f. Cytochrom C | veränderter Wasserfluß [1/m²hbar] | Abnahme d.Wasserflusses |
|---|---|---|---|
| 14 | 100 % | 13 | 7.2 % |
| 17 | 100 % | 16.5 | 3.0 % |
| 13 | 99.6 % | 13 | 0 % |
| 14 | 100 % | 12 | 14.3 % |

## Ansprüche

1. Verfahren zur Oberflächenmodifizierung von Polymer-Membranen um deren Anti-Fouling-Verhalten gegenüber Proteinen zu verbessern, **dadurch gekennzeichnet**, daß man die Polymer-Membranen mit organisch modifizierten Silanen der allgemeinen Formel (I)

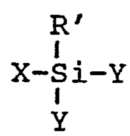 (I),

worin
X = H, Alkyl, Aryl oder Aralkyl, jeweils mit $C_1$ bis $C_{20}$,
R′ = Alkyl, Aryl, Aralkyl, jeweils mit $C_1$ bis $C_{20}$, oder Y, und
Y = OR, OOCR oder NRR″, mit R = Alkyl, Aryl oder Aralkyl, jeweils mit $C_1$ bis $C_{20}$, und R″ = H oder R, bedeuten,
oder mit Gemischen dieser organisch modifizierten Silane (I) beschichtet.

2. Verfahren nach Anspruch 1, **dadurch gekennzeichnet**, daß man die organisch modifizierten Silane der Formel (I) oder deren Gemische vor dem Beschichten der Polymer-Membranen mit einem Lösungsmittel oder einem Lösungsmittelgemisch verdünnt.

3. Verfahren nach Anspruch 1, **dadurch gekennzeichnet**, daß man die Polymer-Membranen mit den unverdünnten organisch modifizierten Silane der Formel (I) oder mit deren unverdünnten Gemischen beschichtet.

4. Verfahren nach Anspruch 2, **dadurch gekennzeichnet**, daß man die organisch modifizierten Silane der Formel(I) oder deren Gemische vor dem Beschichten der Polymer-Membranen mit Petrolether verdünnt.

5. Verfahren nach einem oder mehreren der Ansprüche 1 bis 4, **dadurch gekennzeichnet**, daß man als organisch modifiziertes Silan der Formel (I) das Triethoxysilan verwendet.

6. Verfahren nach einem oder mehreren der Ansprüche 1 bis 5, **dadurch gekennzeichnet**, daß man die organisch modifizierten Silane der Formel (I) bzw. deren Gemische oder die Lösungen der organisch modifizierten Silane bzw. der Gemische auf die Polymer-Membranen einmal oder mehrmals aufsprüht.

7. Verfahren nach einem oder mehreren der Ansprüche 1 bis 5, **dadurch gekennzeichnet**, daß man die organisch modifizierten Silane der Formel (I) bzw. deren Gemische oder die Lösungen der organisch modifizierten Silane bzw. der Gemische auf die Polymer-Membranen einmal oder mehrmals aufstreicht.

8. Verfahren nach einem oder mehreren der Ansprüche 1 bis 5, **dadurch gekennzeichnet**, daß man die Polymer-Membranen in die organisch modifizierten Silane der Formel (I) bzw. in deren Gemische oder in Lösungen der organisch modifizierten Silane der Formel (I) bzw. der Gemische einmal oder mehrmals taucht.

9. Polymer-Membranen, **gekennzeichnet durch** eine Oberflächenmodifizierung gemäß einem Verfahren nach einem oder mehreren der Ansprüche 1 bis 8.